(19) Europäisches Patentamt
European Patent Office
Office européen des brevets

(11) **EP 3 842 140 B1**

(12) **EUROPEAN PATENT SPECIFICATION**

(45) Date of publication and mention
of the grant of the patent:
**28.01.2026 Bulletin 2026/05**

(51) International Patent Classification (IPC):
**B01J 23/745** (2006.01)    **B01J 23/78** (2006.01)
**B01J 29/46** (2006.01)     **B01J 35/77** (2024.01)
**B01J 37/03** (2006.01)     **C07C 1/12** (2006.01)
**C10G 2/00** (2006.01)

(21) Application number: **20217016.3**

(22) Date of filing: **23.12.2020**

(52) Cooperative Patent Classification (CPC):
**B01J 23/745; B01J 23/78; B01J 29/46;**
**B01J 35/77; B01J 37/03; C10G 2/50;** B01J 35/70;
B01J 35/80; B01J 2235/00; B01J 2235/15;
B01J 2235/30

(54) **METHOD FOR CONVERTING CARBON DIOXIDE AND CARBON DIOXIDE CONVERSION METHOD USING A SINGLE CATALYST**

VERFAHREN ZUR UMWANDLUNG VON KOHLENDIOXID UNTER VERWENDUNG EINES EINZELKATALYSATORS

PROCÉDÉ DE CONVERSION DE DIOXYDE DE CARBONE UTILISANT UN CATALYSEUR UNIQUE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **26.12.2019 KR 20190175389**

(43) Date of publication of application:
**30.06.2021 Bulletin 2021/26**

(73) Proprietor: **Research & Business Foundation**
**Sungkyunkwan**
**University**
**Suwon-si, Gyeonggi-do 16419 (KR)**

(72) Inventors:
• **KIM, Jae Hoon**
**Gyeonggi-do (KR)**
• **MUHAMMAD, Kashif Khan**
**Gyeonggi-do (KR)**
• **MUHAMMAD, Irshad**
**Gyeonggi-do (KR)**
• **JO, Heun Tae**
**Gyeonggi-do (KR)**

(74) Representative: **Schmid, Nils T.F.**
**SKM-IP PartGmbB**
**Oberanger 45**
**80331 München (DE)**

(56) References cited:
**WO-A2-2014/111919     US-A1- 2019 016 964**

• **DING FANSHU ET AL: "CO 2 Hydrogenation to Hydrocarbons over Iron-based Catalyst: Effects of Physicochemical Properties of Al 2 O 3 Supports", INDUSTRIAL & ENGINEERING CHEMISTRY RESEARCH, vol. 53, no. 45, 12 November 2014 (2014-11-12), pages 17563 - 17569, XP055802988, ISSN: 0888-5885, DOI: 10.1021/ie5031166**
• **MIRON V. LANDAU ET AL: "Sustainable Production of Green Feed from Carbon Dioxide and Hydrogen", CHEMSUSCHEM, vol. 7, no. 3, 23 February 2014 (2014-02-23), pages 785 - 794, XP055117993, ISSN: 1864-5631, DOI: 10.1002/ cssc.201301181**
• **B. D ROITER: "Phase Equilibria in the Spinel Region of the System FeO-Fe2O3-Al2O3", JOURNAL OF THE AMERICAN CERAMIC SOCIETY, 1 October 1964 (1964-10-01), pages 509 - 511, XP055117943, Retrieved from the Internet <URL:http://onlinelibrary.wiley.com/ store/10.1111/j.1151-2916.1964.tb13799.x/asset/ j.1151-2916.1964.tb13799.x.pdf?v=1& t=hv6k3ayu& s=f5e472eb3f24f5d653a5be17 d26589e20f019d7c> DOI: 10.1111/ j.1151-2916.1964.tb13799.x**

EP 3 842 140 B1

**Description**

**BACKGROUND**

1. **Field**

The present disclosure relates to method using a single catalyst for converting carbon dioxide into useful compounds and a method for conversion of carbon dioxide using the catalyst.

2. **Description of Related Art**

Liquid hydrocarbons such as olefins and paraffins are essential compounds that are widely used as transportation fuels and raw materials for synthesis of many commercial compounds. In industry, liquid hydrocarbons are produced by crude distillation in petroleum refineries. Recently, not only to reduce emission of carbon dioxide (about 400 ppm) by humans into the atmosphere, but also to provide a continuous solution to cope with lack of the fossil fuel reserves, carbon dioxide has been recognized as a potential source for production of not only fuels such as gases and liquid hydrocarbons but also chemical materials such as light olefin, aromatic compound and alcohols. However, carbon dioxide which is inherently thermodynamically very stable and inert may not be hydrogenated by adding a C-C coupling reaction thereto. Thus, carbon dioxide hydrogenation usually produces a product having a high H/C ratio because methane is formed rapidly in the carbon dioxide hydrogenation. This is due to low heat of adsorption of carbon dioxide on a surface of an active catalyst.

To date, carbon dioxide has been converted to $C_1$ compounds (e.g., methanol, methane, formic acid, and carbon monoxide) via several approaches.

Research for converting thermodynamically stable carbon dioxide into liquid hydrocarbon using a metal oxide/zeolite composite catalyst has been attracting attention. Significant progress has been made on that research recently. However, producing a liquid hydrocarbon with a single catalyst without use of an additional C-C coupling agent was still a great challenge. However, a high mechanical barrier and inherent inertness of $CO_2$ ($\Delta Gf° = -396$ kJ/mol) limits the C-C coupling reaction. As a result, selectivity of $C_{5+}$ liquid hydrocarbon was reduced. Each of reverse-water-gas-shift (RWGS, $CO_2 + H_2 \rightarrow CO + H_2O$, $\Delta H_{573K} = 38.0$kJ/mol) and Fischer-Tropsch reaction (FT, $CO + H_2 \rightarrow C_nH_m$, $\Delta H_{573K} = -166.0$ kJ/mol) is an expected strategy for direct production of long-chain hydrocarbons from $CO_2$. Nevertheless, the selectivity of $C_{5+}$ liquid hydrocarbons does not exceed 60%, due to limitation by an Anderson-Schulz-Flory (ASF) distribution. Therefore, despite several attempts to selectively convert $CO_2$ to $C_{5+}$ liquid hydrocarbons, the challenge to design a single type catalyst capable of inhibiting methane formation to produce the $C_{5+}$ liquid hydrocarbons at a high yield and inhibiting excessive hydrogenation to enhance the C-C coupling reaction remains. Currently, the $C_{5+}$ yield ($CO_2$ conversion percentage $\times$ $C_{5+}$ selectivity) due to a single type catalyst remains in a range of 3.0 to 16.1%.

Recently, a concept of a catalyst composite using two types of catalysts (e.g., metal oxide/zeolite composite) has been applied to selectively convert not only $CO_2$ but also syngas to lower olefins ($C_2$ to $C_4$), gasoline ($C_{5+}$), and aromatics. In the metal oxide/zeolite catalyst, the metal oxide converts $CO_2$ to CO via the RWGS reaction, and zeolite (e.g., HZSM-5, beta-zeolite) provides an acidic moiety for the C-C coupling reaction to produce the $C_{5+}$ hydrocarbons and aromatics. Using this metal oxide/zeolite composite concept, considerable progress has been made in the conversion of $CO_2$ to the $C_{5+}$ liquid hydrocarbons at selectivity in a 55 to 80% range (except for CO). However, in some cases, the selectivity of CO is very high (32 to 57%), thus reducing the overall $C_{5+}$ yield to 3.1 to 6.4%. Further, the metal oxide/zeolite composite catalyst had the $C_{5+}$ selectivity greatly varying depending on an approach used to synthesize the metal oxide and the zeolite (e.g., double layer, granule lamination, and mortar mixing), and was very sensitive to formation of coke in pores of the zeolite. A size of the composite catalyst was limited. Control of a spacing between the active sites of the metal oxide and of the zeolite controls sequential diffusion of reaction intermediates and ultimately determines the selectivity of the product, and is by no means trivial. Therefore, it is very important in the field of $CO_2$ hydrogenation to develop a single type and bifunctional catalyst that may effectively promote the RWGS reaction and the subsequent C-C coupling reaction to produce the C5+ liquid hydrocarbons.

In addition to gasoline and diesel range hydrocarbons ($C_{5+}$) and lower olefins ($C_2$ to $C_4$), higher olefins with terminal C=C double bonds (linear $\alpha$-olefins: LAOs) are very important and expensive chemical raw materials. LAOs are a valuable basic component in the production of synthetic lubricants, plasticizers, and common materials (e.g., linear low density polyethylene). Depending on the number of carbons, $C_4$ to $C_8$ LAOs are widely used to produce polyethylene comonomers and low molecular weight fatty acids. $C_6$ to $C_{18}$ LAOs are used to produce plasticizer alcohols, dielectric chemical materials, and surfactants. Long-chain LAOs ($C_{20}$ to $C_{30}$) are widely used to produce dielectric chemical materials (e.g., linear low density polyethylene resins, waxes, and drilling fluids). Therefore, the selective formation of $C_{5+}$ LAOs from $CO_2$ is a very useful and economical technique for producing high value-added compounds in various industrial fields.

WO 2014 111 919 A2 discloses a catalyst and a process for catalytic conversion of carbon dioxide-containing gas and hydrogen streams to hydrocarbons. Miron v. Landau et al. [CHEMSUSCHEM, vol. 7, no. 3, 23 February 2014, pages 785-794, ISSN: 1864-5631, DOI: 10.1002/cssc.201301181] describe a sustainable production of green feed from carbon dioxide and hydrogen.

**SUMMARY**

**1. Technical purposes**

This Summary is provided to introduce a selection of concepts in a simplified form that are further described below in the Detailed Description. This Summary is not intended to identify all key features or essential features of the claimed subject matter, nor is it intended to be used alone as an aid in determining the scope of the claimed subject matter.

What the applicant wants to achieve in accordance with the present disclosure is a bifunctional salt promoted with $FeAlO_x$ which had high selectivity to $C_{5+}$ LAOs without a C-C coupling catalyst such as zeolite, and promotes the formation of the $C_{5+}$ hydrocarbons at the high yield. The $FeAlO_x$ catalyst has limited selectivity (7.2%) to CO and selectivity (12.1%) to $CH_4$ at 36.8% $CO_2$ conversion percentage, and realizes a high $C_{5+}$ yield of 19.7% (including CO). In addition, under hydrogen-deficient conditions, the selectivity thereof to $C_{5+}$ is very high, that is, 77.0% despite the $CO_2$ conversion percentage of 20.2%. The selectivity thereof to $C_{5+}$ hydrocarbons exceeds the highest value as predicted in ASF as the model of contribution to the FT reaction. A selectivity 52.4% thereof to overall LAOs is achieved when the carbon distribution (except for CO) is considered. This value is the highest value as reported to date. The catalyst according to the present disclosure exhibited very high stability under stream for up to 450 hours while causing negligible change in the product composition. This supports the tremendous potential for practical application thereof.

A purpose of the present disclosure is to provide a single catalyst that may increase the conversion percentage of carbon dioxide, and increase the yield of $C_{5+}$ liquid hydrocarbons and long-chain $\alpha$-olefins from the carbon dioxide, and to provide a method for converting carbon dioxide using that catalyst.

Purposes in accordance with the present disclosure are not limited to the above-mentioned purpose. Other purposes and advantages in accordance with the present disclosure as not mentioned above may be understood from following descriptions and more clearly understood from embodiments in accordance with the present disclosure. Further, it will be readily appreciated that the purposes and advantages in accordance with the present disclosure may be realized by features and combinations thereof as disclosed in the claims.

**2. Technical solutions**

One aspect of the present disclosure provides a carbon dioxide conversion method using a catalyst with two active sites.

One aspect of the present disclosure uses a catalyst having a composition of $Fe_xAl_yO_z$, wherein the catalyst may have two active sites including acidic and basic sites. The catalyst serves to hydrogenate the carbon dioxide and convert the carbon dioxide to $C_{5+}$ liquid hydrocarbons and $C_{5+}$ linear $\alpha$-olefins at high yield. In the composition of the catalyst, x may be a real number of 0.1 or greater to 0.8 or smaller, and y may be a real number of 0.2 or greater to 0.9 or smaller, and z is in a range in which the catalyst may form iron oxide and aluminum oxide, and may be a real number of 0.5 or greater to 3 or smaller.

In one implementation, in the catalyst, a ratio of Fe:Al may be 50:50. As the percentage of iron increases to 50, a percentage at which hydrocarbons are resorbed thereon increases, so that the selectivity to $C_{5+}$ hydrocarbons increases. However, when the percentage of the aluminum exceeds 50, the selectivity to $C_{5+}$ hydrocarbons decreases slightly. A Fe:Al ratio range of 10:90 to 80:20 may be a range in which the conversion percentage and the selectivity to $C_{5+}$ hydrocarbons vary at an acceptable level. The most ideal Fe:Al ratio in terms of both of the carbon dioxide conversion and the selectivity to the product may be the 50:50 ratio.

According to the invention, the catalyst includes a crystallized $Fe_2O_3$ phase, which may be a basic site in the carbon dioxide conversion process. The $Fe_2O_3$ phase may act as a site where carbon dioxide is adsorbed and hydrocarbons are formed during the carbon dioxide conversion. In the catalyst, the $Fe_2O_3$ phase may be activated by being calcined at 400 to 1000°C. Preferably, the $Fe_2O_3$ phase may activated by being fired at 400 to 650°C.

In one implementation, the catalyst may further include an alkali metal. The alkali metal may include at least one among sodium (Na), potassium (K), rubidium (Rb), and cesium (Cs). In the catalyst, an amount of the alkali metal may be in a range of 0.5 to 10 wt% with respect to a total weight of the catalyst. When the amount of the alkali metal is less than 0.5 wt%, a $CO_2$ conversion ratio may be decreased because an amount of carbon dioxide absorbed to a basic site of the alkali metal is decreased. When the amount of the alkali metal is larger than 10 wt%, a selectivity may be decreased because RWGS active sites and FT active sites are decreased.

According to the invention, the catalyst contains amorphous aluminum oxide which corresponds to the acidic site in the process of carbon dioxide conversion. The aluminum oxide is a site where hydrogen desorption occurs in the process of the carbon dioxide conversion. The amorphous aluminum oxide may re-absorb $C_2$ to $C_4$ formed during the conversion process and thus plays an important role in the formation of $C_{5+}$ hydrocarbons. The amorphous aluminum oxide may be fired at 400 to 1000°C to act as the acidic site. Preferably, the amorphous aluminum oxide may be calcined at 400 to 650°C to act as the acidic site.

According to the invention, the aluminum oxide along with the $Fe_2O_3$ phase are contained in the catalyst. The aluminum oxide and the $Fe_2O_3$ phase may be adjacent to each other.

In one implementation, the catalyst may be combined with zeolite to form a composite which may be used as a carbon dioxide conversion catalyst. A conventional zeolite composite catalyst as the conventional carbon dioxide conversion catalyst was used to increase the selectivity to $C_{5+}$ hydrocarbons. The catalyst according to the present disclosure may be combined with the zeolite to improve the carbon dioxide conversion and the product yield.

The invention of the present disclosure provides a carbon dioxide conversion method using the catalyst, the method including reacting the catalyst with hydrogen gas to absorb hydrogen thereon, and injecting a mixture gas of carbon dioxide and hydrogen such that the mixture gas reacts with the hydrogen adsorbed catalyst. The carbon dioxide conversion method may be a method in which carbon dioxide is converted to compounds via a C-C coupling reaction by RWGS synthesis and FT synthesis on the catalyst.

In one implementation of the conversion method, the mixing ratio of $H_2$:$CO_2$ may be 3:1 to 1:3.

In one implementation of the conversion method, the gas mixture of the carbon dioxide and the hydrogen may be injected at a space velocity of a range of 2000 to 10000 mL/g·h.

In one implementation of the conversion method, the reaction pressure of the $H_2/CO_2$ mixture gas may be in a range of 1.5 to 3.5 MPa.

In one implementation of the conversion method, the conversion method may be carried out at 300 to 500°C, preferably, 330 to 450 °C.

In one implementation of the conversion method, the conversion method may result in selectively production of $C_{5+}$ liquid hydrocarbons and $C_{5+}$ linear $\alpha$-olefins.

### 3. Technical effects

Effects in accordance with the present disclosure may be as follows but may not be limited thereto.

According to the present disclosure, the carbon dioxide conversion percentage, and the yield of $C_{5+}$ liquid hydrocarbons and $C_{5+}$ long chain $\alpha$-olefins may be improved using only the single catalyst, without the use of the zeolite composite catalyst conventionally used to increase the yield.

In addition to the effects as described above, specific effects in accordance with the present disclosure will be described together with the detailed description for carrying out the disclosure.

### BRIEF DESCRIPTIONS OF DRAWINGS

FIG. 1 shows HT-TEM, and XRD results and a Mössbauer spectrum of a catalyst of Example 1 according to a carbon dioxide conversion step.

FIG. 2 is a UHV-XPS result of aluminum.

FIG. 3 is an XRD result based on a ratio of Fe:Al.

FIG. 4 is a TEM result of a reduced catalyst.

FIG. 5 is a TEM result of a spent catalyst.

FIG. 6 is data evaluating an effect of amorphous aluminum on a $CO_2$ conversion percentage and selectivity to products.

FIG. 7 shows a result of a $H_2$-TPD (temperature-programmed desorption) test.

FIG. 8 shows a result of a $NH_3$-TPD (temperature-programmed desorption) test.

FIG. 9 shows a result of a $CO_2$-TPD (temperature-programmed desorption) test.

FIG. 10 shows a result of a nitrogen ($N_2$) adsorption-desorption isotherm test.

FIG. 11 shows a result of NAP-XPS (near-ambient pressure X-ray photoelectron spectroscopy) of the catalyst of Example 1.

FIG. 12 is a result of UHV-XPS (ultra high vacuum X-ray photoelectron spectroscopy) of the catalyst of Example 1.

FIG. 13 is an XAS (X-ray adsorption spectroscopy) result of a reduced catalyst.

FIG. 14 shows a result of a carbon dioxide conversion test at various hydrogen : carbon dioxide ratios using the catalyst of Example 1.

FIG. 15 shows a result of a carbon dioxide conversion test at various pressures using the catalyst of Example 1.

FIG. 16 shows a result of a carbon dioxide conversion test at various space velocities using the catalyst of Example 1.
FIG. 17 shows a carbon dioxide conversion test result of various catalysts including the catalyst of Example 1.
FIG. 18 shows comparisons between $CO_2$ conversion percentages of the catalyst of Example 1 and conventional catalysts, and between selectivities of the catalyst of Example 1 and conventional catalysts to $C_{5+}$.
FIG. 19 shows carbon dioxide conversion test results of the catalyst of Example 1 and a zeolite composite catalyst.
FIG. 20 shows a result of evaluating reproducibility of the catalyst of Example 1.
FIG. 21 shows a result of evaluating stability of the catalyst of Example 1.

## DETAILED DESCRIPTIONS

For simplicity and clarity of illustration, elements in the figures are not necessarily drawn to scale. The same reference numbers in different figures represent the same or similar elements, and as such perform similar functionality. Further, descriptions and details of well-known steps and elements are omitted for simplicity of the description. Furthermore, in the following detailed description of the present disclosure, numerous specific details are set forth in order to provide a thorough understanding of the present disclosure. However, it will be understood that the present disclosure may be practiced without these specific details. In other instances, well-known methods, procedures, components, and circuits have not been described in detail so as not to unnecessarily obscure aspects of the present disclosure.

Examples of various embodiments are illustrated and described further below. It will be understood that the description herein is not intended to limit the claims to the specific embodiments described. On the contrary, it is intended to cover alternatives, modifications, and equivalents as may be included within the spirit and scope of the present disclosure as defined by the appended claims.

The terminology used herein is for the purpose of describing particular embodiments only and is not intended to limit the present disclosure. As used herein, the singular forms "a" and "an" are intended to include the plural forms as well, unless the context clearly indicates otherwise. It will be further understood that the terms "comprises", "comprising", "includes", and "including" when used in this specification, specify the presence of the stated features, integers, operations, elements, and/or components, but do not preclude the presence or addition of one or more other features, integers, operations, elements, components, and/or portions thereof. As used herein, the term "and/or" includes any and all combinations of one or more of the associated listed items. Expression such as "at least one of" when preceding a list of elements may modify the entire list of elements and may not modify the individual elements of the list.

Unless otherwise defined, all terms including technical and scientific terms used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

As used herein, the terms "substantially," "substantial," "approximately," and "about" are used to denote and account for small variations. When used in conjunction with an event or circumstance, the terms can refer to instances in which the event or circumstance occurs precisely, as well as instances in which the event or circumstance occurs to a close approximation. For example, when used in conjunction with a numerical value, the terms can refer to a range of variation of less than or equal to ±10% of that numerical value, such as less than or equal to ±5%, less than or equal to ±4%, less than or equal to ±3%, less than or equal to ±2%, less than or equal to ±1%, less than or equal to ±0.5%, less than or equal to ±0.1%, or less than or equal to ±0.05%. For example, a first numerical value can be "substantially" the same as a second numerical value if the first numerical value is within a range of variation of less than or equal to ±10% of the second numerical value, such as less than or equal to ±5%, less than or equal to ±4%, less than or equal to ±3%, less than or equal to ±2%, less than or equal to ±1%, less than or equal to ±0.5%, less than or equal to ±0.1%, or less than or equal to ±0.05%. For example, "substantially" parallel can refer to a range of angular variation relative to 0° that is less than or equal to ±10°, such as less than or equal to ±5°, less than or equal to ±4°, less than or equal to ±3°, less than or equal to ±2°, less than or equal to ±1°, less than or equal to ±0.5°, less than or equal to ±0.1°, or less than or equal to ±0.05°.

Additionally, amounts, ratios, and other numerical values are sometimes presented herein in a range format. It can be understood that such range formats are used for convenience and brevity, and should be understood flexibly to include not only numerical values explicitly specified as limits of a range, but also all individual numerical values or subranges encompassed within that range as if each numerical value and sub-range is explicitly specified.

In a "$FeAlO_x$-number" as used herein, a ratio of Fe and Al is determined by "the number" appended to "$FeAlO_x$" (for example, in $FeAlO_x$-5, Fe 50at% and Al 50at%; and in $FeAlO_x$-8, Fe 80at% and Al 20at%).

## Example 1: FeAlO$_x$-5 catalyst synthesis

Iron-aluminum oxide ($FeAlO_x$) catalysts having various Fe/Al ratios were synthesized using a co-precipitation method while controlling the ratio of iron and aluminum salts.

1) FeAlO$_x$-5 synthesis process

Iron(III) nitrate nonahydrate (Fe(NO$_3$)$_3$·9H$_2$O, 99.9%, Alfa Aesar, USA) 10g and aluminum(III) nitrate nonahydrate (Al(NO$_3$)$_3$·9H$_2$O, 99.9%, Alfa Aesar, USA) 10 g were dissolved in 150 mL of DDI (distilled and deionized) water (solution ①). A solution in which 11 g of sodium carbonate (Na$_2$CO$_3$, 99.98%, Sigma Aldrich, USA) was dissolved in 53mL DDI water was used as a precipitant. The Na$_2$CO$_3$ aqueous solution was added dropwise to the solution ① while stirring at 80°C. During the dropwise addition of the precipitant, a dark brown precipitate was formed. The solution mixture was stirred continuously for 12 hours with a bar magnet while maintaining pH 8. Then, the mixture was aged at room temperature for 6 hours. When most of the precipitate was formed, the precipitate was filtered using Whatman #2 filter paper. After the filtration, the precipitate remaining on the filter paper was washed with 300 mL DDI water. Then, the filtered substance was placed in a drying oven at 80°C for 12 hours, and then, was calcined at 600°C for 6 hours in a static air condition.

### Example 2: FeAlO$_x$-5/HZSM-5 composite catalyst synthesis

In order to synthesize the FeAlO$_x$-5/HZSM-5 composite catalyst, aluminum-ZSM-5 (NH$_4$-ZSM-5) (Alfa Aesar, product #45882) with 80 of a Si/Al ratio was calcined to 550°C at 2°C/min under an airflow of 50 mL/min and thus was converted to NH$_4$-ZSM-5 (HZSM-5) in a hydrogenated form. The calcined HZSM-5 powders were physically mixed with FeAlO$_x$-5 at a mass ratio of 1:1 and with 3 mL silica particles (Fischer Chemical, S/0365/60) in a vial. The catalyst and silica mixture were shaken throughout and were homogenized and then, was transferred to a reactor. The catalyst mixture was reduced at 450°C and 3.5 MPa for 10 hours under H$_2$ at a rate of 30 mL/min before reaction.

### Comparative Example

Comparative examples were prepared for comparison with the FeAlO$_x$ catalyst and the FeAlO$_x$-5/HZSM-5 composite catalyst.

1) Synthesis of Na-Fe$_3$O$_4$

The magnetic iron oxide-promoted sodium (Na-Fe$_3$O$_4$) catalyst was prepared using a conventionally reported method. Briefly, while 31.6 g of iron(III) chloride (FeCl$_3$·6H$_2$O, 98%, Alfa Aesar) and 12.54 g of iron(II) chloride (FeCl$_2$·6H$_2$O, 99.9%, Sigma Aldrich) were continuously mixed and stirred at room temperature (23±0.5□), 150 mL DDI water was added thereto, thus, to form a homogenous solution. Thereafter, 5.1 mL of a concentrated hydrochloric acid (12.1 mol/L) solution was added dropwise to the homogeneous solution at room temperature (23±0.5°C) until a transparent solution was formed. Thereafter, a concentrated aqueous sodium hydroxide (NaOH, 1.5 mol/L) aqueous solution was added dropwise to the solution mixture at 60°C while continuously stirring the mixture. As the NaOH solution was added thereto, a black precipitate began to be formed. During the formation of the precipitate, a pH of the solution was maintained at 10. The mixture was stirred continuously for 1 hour. The black precipitate was separated therefrom using a magnet, and was washed with 800 mL of DDI water. The synthesized catalyst was dried at 60°C for 12 hours.

2) Synthesis of catalysts with various ratios

The FeAlO$_x$ catalysts with various Fe/Al ratios were synthesized while controlling the amounts of Fe(NO$_3$)$_3$·9H$_2$O and Al(NO$_3$)$_3$·9H$_2$O in the solution ①. The conditions except for the amounts of Fe(NO$_3$)$_3$·9H$_2$O and Al(NO$_3$)$_3$·9H$_2$O in the synthesis process are the same as those applied to the synthesis of FeAlO$_x$-5. To synthesize FeAlO$_x$-10 (Fe 100%; Al 0%), 10 g of Fe(NO$_3$)$_3$·9H$_2$O was dissolved in 100mL DDI water, and 5.3 g of Na$_2$CO$_3$ dissolved in 25 mL of DDI water was used as a precipitant. To synthesize FeAlO$_x$-8 (Fe 80%; Al 20%), 16 g of Fe(NO$_3$)$_3$·9H$_2$O and 4g of Al(NO$_3$)$_3$·9H$_2$O were dissolved in 100mL DDI water. When a transparent solution was formed, 11 g of Na$_2$CO$_3$ dissolved in 52mL DDI water was used as a precipitant. FeAlO$_x$-0 (Fe 0%; Al 100%) was synthesized by dissolving Al(NO$_3$)$_3$·9H$_2$O in 100mL DDI water. Thereafter, 5.7 g of Na$_2$CO$_3$ dissolved in 27mL DDI water was used as a precipitant. In addition, catalysts with varying composition ratios (FeAlO$_x$-2, FeAlO$_x$-1) were prepared while varying the weights of Fe(NO$_3$)$_3$·9H$_2$O and Al(NO$_3$)$_3$·9H$_2$O.

### Example 3: carbon dioxide conversion process

The carbon dioxide conversion test was performed using the catalyst prepared in Example 1.

1) Formation of reduced catalyst

The catalyst of Example 1 was reduced at 450°C and 3.5 MPa $H_2$ pressure under $H_2$ at 30 mL/min for 10 hours. At this time, a crystalline $Fe_2O_3$ was reduced to $Fe_3O_4$, and hydrogen was adsorbed to an $AlO_x$ site.

2) C-C coupling reaction via RWGS and FT synthetic pathways

2000mL/h·g ($CO_2$ : 1000mL/h·g, $H_2$ : 1000mL/h·g) of $H_2/CO_2$ in a 1:1 ratio was injected to the reactor at 330°C and 3.5 MPa such that the reduced catalyst was reacted therewith for 80 hours on stream. CO formed via RWGS of $CO_2$ forms hydrocarbons ($C_2$ to $C_4$) via a C-C coupling reaction using the FT synthesis pathway, and a portion of $Fe_3O_4$ is converted to $Fe_5C_2$.

3) $C_{5+}$ liquid hydrocarbon and linear $\alpha$-olefin formation via C-C coupling reaction

The $C_2$ to $C_4$ hydrocarbon formed in the 2) is re-adsorbed to the amorphous $AlO_x$ site to form $C_{5+}$ liquid hydrocarbon and a $C_{5+}$ linear $\alpha$-olefin via a C-C coupling reaction. After the reaction, the spent catalyst was collected and analyzed.

The catalysts in the Example are intended for the active conversion of $CO_2$ to long chain $\alpha$-olefins (LAOs) and $C_{5+}$ liquid hydrocarbons at high $CO_2$ conversion percentage and high selectivity to $C_{5+}$. Therefore, in order to investigate the characteristics of the catalyst causing such conversion, a following test was performed.

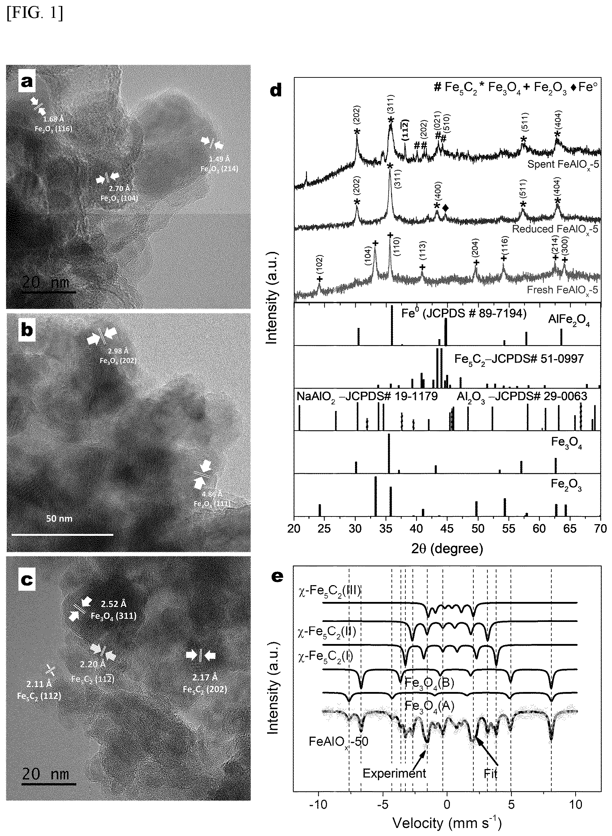

FIG. 1 shows the HR-TEM and XRD results of $FeAlO_x$-5 pure phase (a, fresh, the catalyst of Example 1), reduced catalyst (b, reduced catalyst of Example 1) and spent catalyst (c, reaction-completed catalyst of Example 1). The results in FIG. 1 show that the catalyst of Example 1 has two different phases, that is, a crystallized $Fe_2O_3$ phase and an amorphous $AlO_x$ phase. The catalyst of Example 1 includes a hematite ($Fe_2O_3$) phase with (104), (110), (116), and (214) faces. In the catalyst of Example 1, no peaks related to the crystallized $Al_2O_3$ phase were found in the XRD pattern thereof. Inductively coupled plasma light emission spectroscopy (ICP-OES) shows that the amounts of Fe and Al are 21.8% and 21.1wt%, respectively, in the catalyst of Example 1, thus indicating that a phase including Al is sufficiently present therein.

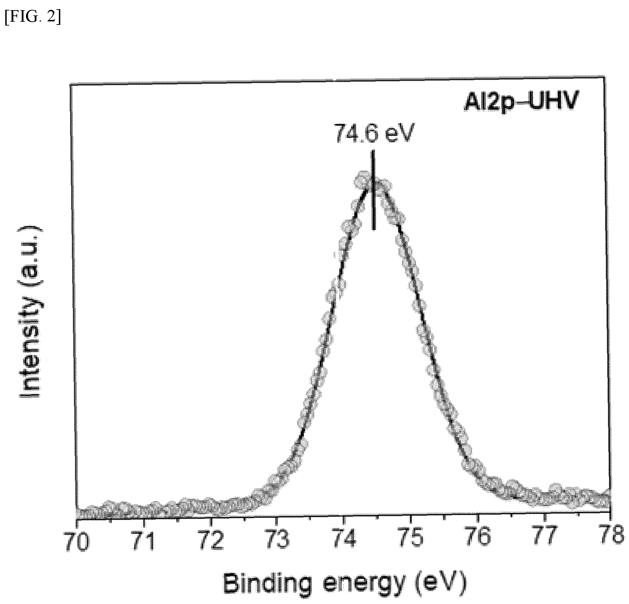

In order to test the chemical properties of the Al-including phase, ultra-high vacuum X-ray photoelectron spectroscopy (UHV-XPS) analysis was performed. FIG. 2 shows a corresponding high-resolution Al 2p profile. A peak at the binding energy of 74.6 eV indicates that amorphous aluminum oxide exists in the catalyst of Example 1. This indicates that the catalyst of Example 1 is substantially composed of crystallized $Fe_2O_3$ and amorphous aluminum oxide phase.

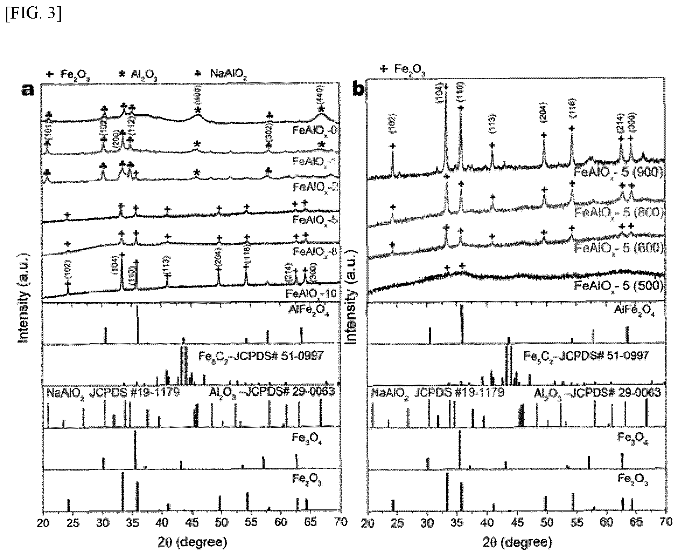

Further, in FIG. 3 showing the XRD results based on the Fe/Al ratio, the catalysts of the comparative examples ($FeAlO_x$-1 and $FeAlO_x$-2) having a high Al content exhibit 45.8° (400) and 66.8° (440) values at $2\theta$, respectively and thus have the peaks similar to that of the crystallized $Al_2O_3$ (JCPDS No. 29-0063). This means that the catalyst including a large amount of Al has a large amount of crystallized $Al_2O_3$ phase (FIG. 3(a)). Further, $NaAlO_2$ crystal phase (JCPDS No. 19-1179) was also found in an aluminum-rich catalyst. This indicates that Al and Na form an oxidized bimetallic phase.

(a) in FIG. 3 shows the XRD results of catalysts calcined at 600□ for 6 hours, while (b) in FIG. 3 shows the XRD results of catalysts subjected to calcining at various calcining temperatures in a range of 500 to 900□. As shown in (b) in FIG. 3, despite the high temperature of 900□, the formation of the crystallized $Al_2O_3$ phase was not found in the catalyst of Example 1. To the contrary, the crystallized $Fe_2O_3$ increases with the increasing temperature. This indicates that in the catalyst of Example 1, the $Fe_2O_3$ phase inhibits the crystallization of $Al_2O_3$. In the catalyst of Example 1 calcined at 600°C, a crystal size of $Fe_2O_3$ was calculated on all major planes ((102), (104), (110), (204), (116), (214), and (300)) using a Scherrer's equation. A calculated value was 25.3 nm. The crystal sizes of the catalysts as prepared while varying the ratio of Fe/Al are shown in Table 1.

[Table 1]

| Catalysts | BET surface area ($m^2g^{-1}$) | Pore volume ($cm^3g^{-1}$) | Average mesopore diameter (nm) | $V_m$ ($cm^3$(STP) $g^{-1}$) | Crystal size (nm) |
|---|---|---|---|---|---|
| $FeAlO_x$-0 | 43.0 | 0.30 | 28.0 | 9.9 | 13.8 |
| $FeAlO_x$-1 | 40.9 | 0.24 | 23.5 | 9.4 | 19.6 |
| $FeAlO_x$-2 | 88.0 | 0.37 | 16.7 | 20.0 | 19.9 |
| $FeAlO_x$-5 (catalyst of Example 1) | 35.6 | 0.28 | 31.7 | 8.2 | 25.3 |
| $FeAlO_x$-5 (Reduced Catalyst) | 63.4 | 0.16 | 10.0 | 14.6 | 20.7 |

(continued)

| Catalysts | BET surface area ($m^2g^{-1}$) | Pore volume ($cm^3g^{-1}$) | Average mesopore diameter (nm) | $V_m$ ($cm^3$(STP) $g^{-1}$) | Crystal size (nm) |
|---|---|---|---|---|---|
| $FeAlO_x$-5 (Spent Catalyst) | 23.9 | 0.14 | 24.8 | 5.5 | 23.9 |
| $FeAlO_x$-8 | 50.5 | 0.18 | 14.2 | 11.6 | 23.0 |
| $FeAlO_x$-10 | 115.0 | 0.10 | 3.5 | 26.5 | 49.3 |

In Table 1, the catalysts other than the reduced catalyst and the spent catalyst were calcined with air at 600°C and 0.1 MPa for 6 hours and then tested. In the reduced catalyst, the hematite phase ($Fe_2O_3$) was converted to the magnetite ($Fe_3O_4$) phase. This was identified based on XRD peaks at 30.1° (202), 35.5° (311), 43.1° (400), 57.0° (511), and 62.6° (404). The XRD pattern of the reduced catalyst also indicates presence of an $\alpha$-Fe phase (JPCDS No. 89-7194) based on a 44.7° peak. This indicates that activity of the catalyst may be improved for $CO_2$ hydrogenation. This is because metallic iron is rapidly converted into $Fe_5C_2$ and $Fe_3O_4$ phases under this test condition. In the reduced catalyst, the crystal size of $Fe_3O_4$ phase was slightly reduced to 20.7nm compared to that of $Fe_2O_3$ (25.3nm) in the catalyst of Example 1.

(c) and (d) in FIG. 1 show the XRD pattern of the spent catalyst and the peaks related to iron carbide ($\chi$-Fe5C2, JCPDS, 51-0997). This result indicates that Hägg iron carbide may enhance the C-C coupling reaction via FT synthesis (Fischer Tropsch reaction) to actively mitigate $CH_4$ formation. Recent cyclic spin polarization density functional theory (DFT) studies have shown that due to the high activation barrier of $CH_4$ and the active participation in the coupling of compounds including C-CH and CH-CH in the formation of long-chain hydrocarbons, the $CH_4$ formation may be effectively inhibited. The XRD pattern of the spent catalyst strongly exhibits a signal of a high Miller index (510) face, which indicates the abundance of this face. The (510) face may play a dominant role in exposing the surface area of $\chi$-Fe5C2 at a maximum degree due to low surface energy thereof. Thus, the presence of the (510) face demonstrates the high activity of the catalyst of Example 1 in hydrogenating $CO_2$.

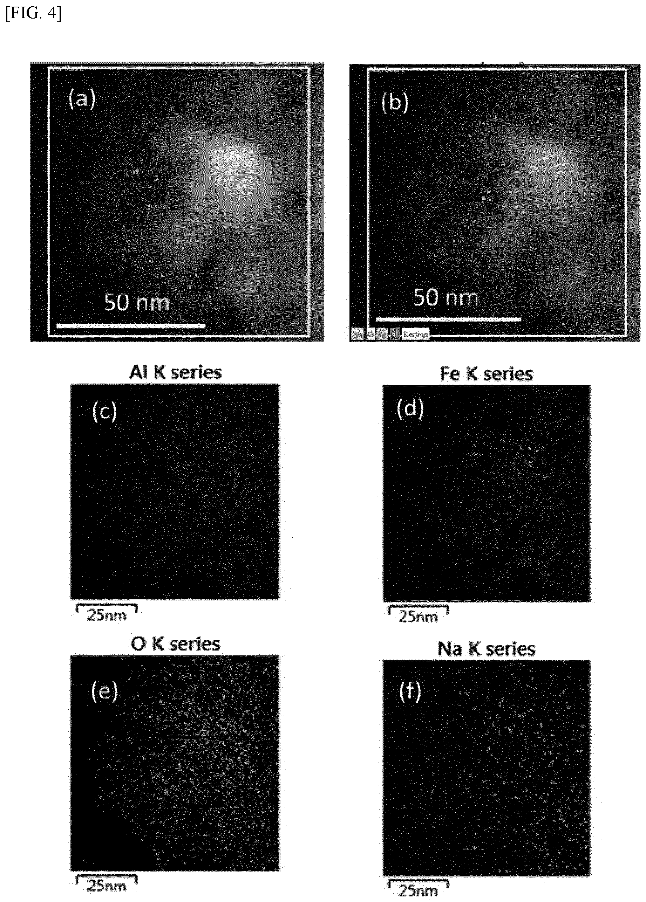

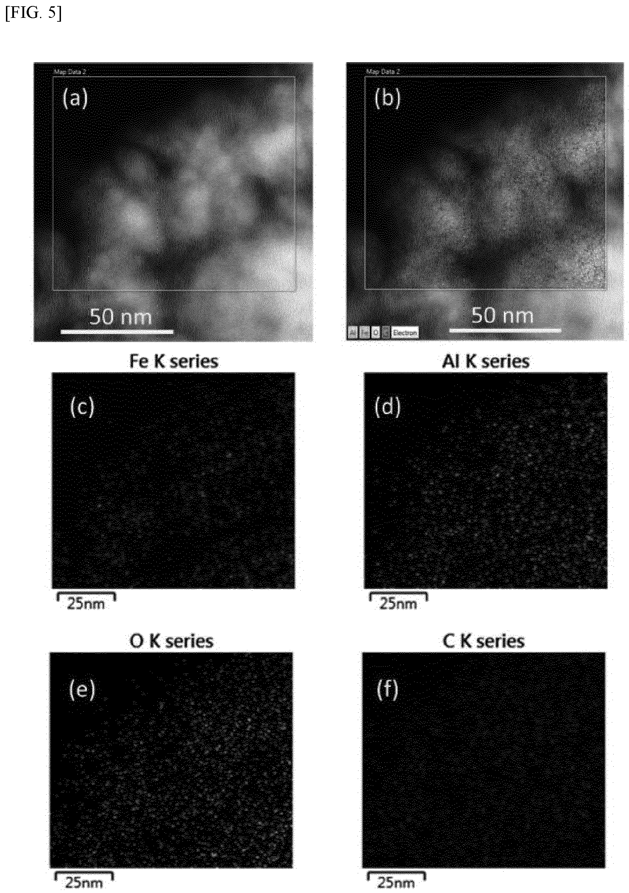

A compound including iron and aluminum oxide was distributed in a uniform manner in the reduced catalyst and the spent catalyst. FIG. 4 is a TEM image of the reduced catalyst. FIG. 5 is a TEM image of the spent catalyst. In order to quantify a phase of active iron in the $CO_2$ hydrogenation, the spent catalyst was analyzed using Mössbauer spectroscopy. A result is shown in FIG. 1(e) and Table 2.

[Table 2]

| Catalysts | Mössbauer factors | | | | |
|---|---|---|---|---|---|
| | Types of phase | IS(mm/s) | QS(mm/s) | Hhf(kOe) | Spectral distribution |
| Spent Catalyst | $Fe_3O_4$(A) | 0.26 | -0.05 | 489 | 13.00 |
| | $Fe_3O_4$(B) | 0.67 | 0.05 | 460 | 22.28 |
| | $Fe_5O_2(\square)$ | 0.17 | 0.08 | 182 | 26.28 |
| | $Fe_5O_2(\square)$ | 0.25 | 0.06 | 217 | 22.00 |
| | $Fe_5O_2(\square)$ | 0.19 | 0.16 | 108 | 16.44 |

In most of the spent catalysts, two phases, that is, $Fe_3O_4$ (RWGS reaction result) and $\chi$-$Fe_5C_2$ (C-C coupling reaction result) coexist, which have active phases dispersed in the spent catalyst. The spectrum of the spent catalyst includes non-equivalent iron phases of $Fe_3O_4$-A (including tetrahedral $Fe^{3+}$ ions) and $Fe_3O_4$-B (including octahedral position $Fe^{3+}$ and $Fe^{2+}$ ions). This is the most optimal model of the iron on $Fe_3O_4$. The most common phase in the spent catalyst is $\chi$-$Fe_5C_2$, which includes three different non-equivalent phases and thus accounts for 64.7%. $Fe_3O_4$ (35.3%) is the second most common phase in the spent catalyst. These results indicate that excellent activity was imparted to the catalyst of Example 1 due to RWGS and following C-C coupling reaction on the catalyst surface.

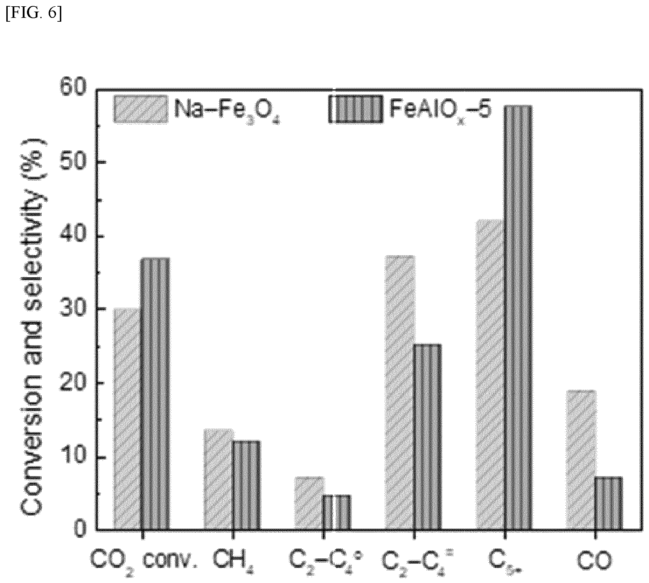

FIG. 6 compares the catalyst performances of the catalyst of Example 1 and Na-$Fe_3O_4$ as related to the effect of the amorphous $AlO_x$ phase on $CO_2$ hydrogenation and selectivity to products. The experiment was performed at 330°C and 3.5 MPa at a ratio of $H_2/CO_2$ of 1:3 and using 4000mL·g-1·h-1 ($CO_2$ 1000mL/g·h, $H_2$ 3000mL/g·h). The catalyst of Example 1 exhibited higher selectivity (57.6%, except for CO) to $C_{5+}$ than Na-$Fe_3O_4$ (42.0%) did. This means that the catalyst of Example 1 is more advantageous in producing the long chain hydrocarbons. In addition, the above result proves that the amorphous $AlO_x$ may provide an effective active site for re-adsorption of $C_2$ to $C_4$ olefins. This may provide a platform to additionally perform C-C coupling to improve selectivity to $C_6$ to $C_{12}$ LAOs. In addition, as the selectivity to CO is higher, the more additional FT synthesis must be performed to improve an overall yield of the liquid hydrocarbon. In this connection, the catalyst of Example 1 has a lower selectivity to CO, compared to that of Na-$Fe_3O_4$ to CO (19.0%). This is advantageous for producing liquid hydrocarbons.

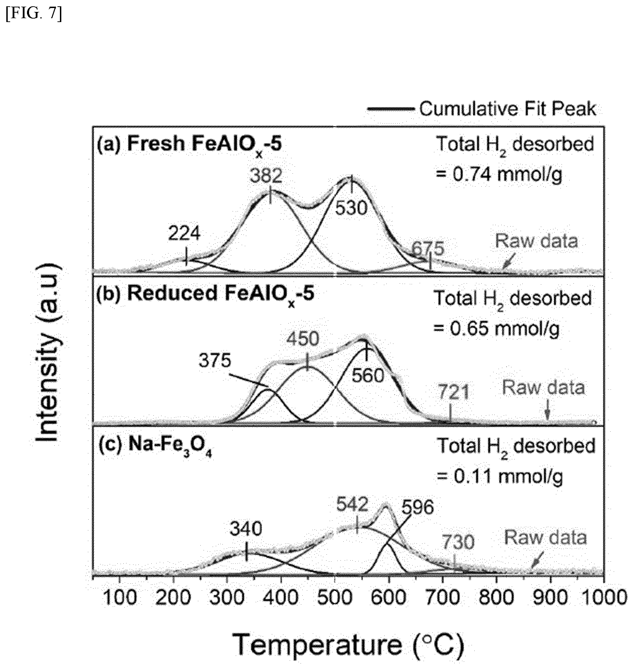

FIG. 7 shows the $H_2$-TPD profiles for (a) the catalyst of Example 1 (fresh), (b) reduced catalyst, and (c) Na-$Fe_3O_4$. The profile is intended to further investigate the unique properties of $FeAlO_x$ catalyst. To this end, $H_2$-TPD analysis based on temperature change is performed to examine possibility of hydrogen spillover. When the hydrogen spillover is performed well, availability of $H_2$ adsorbed on the surface of the catalyst increases. This may be used for $CO_2$ hydrogenation via FT synthesis in the process of producing the long chain hydrocarbons at a high-yield. In FIG. 7, the $H_2$-TPD profile is composed of a low temperature absorption region (≥400°C) and a high temperature absorption region (401 to 800°C). A low temperature absorption peak is related to weak hydrogen desorption on the surface of the catalyst, while a high temperature absorption peak is related to the hydrogen spillover or hydrogen desorption beneath the surface and corresponds to strong desorption. It may be identified that in (a) and (b) in which the amorphous AlOx phase is present, $H_2$ spillover is improved. Detailed data thereof are shown in Table 3.

[Table 3]

| Catalysts | Total desorbed $H_2$ (mmol/g) | Desorption temperature range (mmol/g) | |
| --- | --- | --- | --- |
| | | 100 to 400□ (Weak desorption) | 401 to 800□ (Strong desorption) |
| Catalyst of Example 1 | 0.74 | 0.34 | 0.40 |
| Reduced catalyst | 0.65 | 0.38 | 0.27 |
| $FeAlO_x$-10($Fe_2O_3$) | 0.11 | 0.03 | 0.09 |
| $FeAlO_x$-0($Al_2O_3$) | 0.54 | - | 0.54 |
| Na-$Fe_3O_4$ | 0.11 | 0.03 | 0.08 |

In the catalyst of Example 1 and the reduced catalyst, the ranges of the amount of the hydrogen spillover thereof were similar to each other and the range was 0.27 to 0.40 mmol/g. The total desorbed hydrogen amount was in a range of 0.65 to 0.74 mmol/g. In $FeAlO_x$-0 (pure $Al_2O_3$), there was no evidence of weak hydrogen desorption, but the total desorbed hydrogen amount was 0.54 mmol/g. To the contrary, the hydrogen spillover in Na-$Fe_3O_4$ exhibited an extremely small desorbed-hydrogen amount in the high temperature range. This means that the pure iron-based Na-$Fe_3O_4$ catalyst is not effective in inducing the hydrogen spillover. When the above findings are taken together, an important factor contributing to the hydrogen spillover in the $FeAlO_x$ catalyst is the presence of the amorphous $AlO_x$ phase. Therefore, it may be identified that the catalyst of Example 1 and the reduced catalyst have high heat of hydrogen adsorption and improved hydrogen spillover effect due to the amorphous $AlO_x$ phase and thus are advantageous in converting $CO_2$ to LAOs.

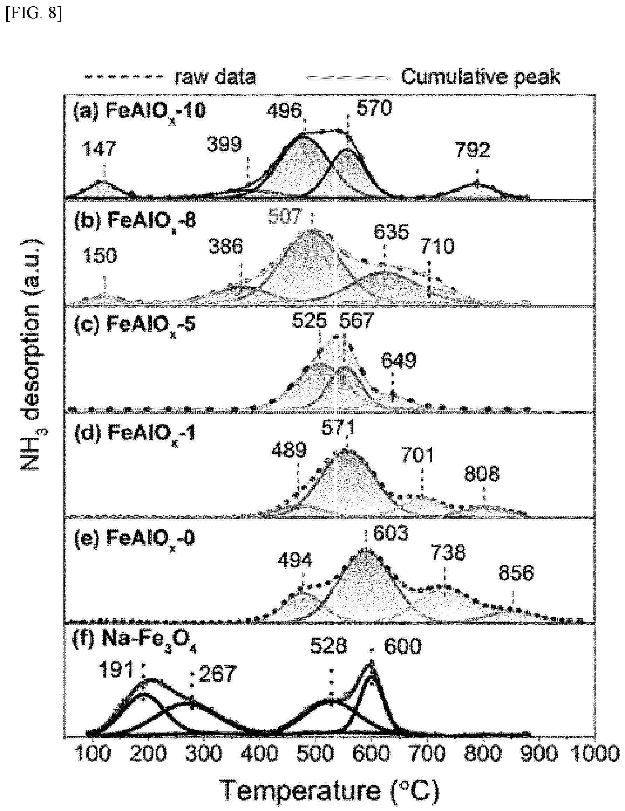

FIG. 8 shows the $NH_3$-TPD profile of the catalyst of Example 1 and Comparative Examples. The profile in FIG. 8 is intended to identify that the presence of the amorphous $AlO_x$ phase improves the acid strength of the $FeAlO_x$ catalyst. To this end, ammonia desorption ($NH_3$-TPD) analysis based on a temperature was performed. Detailed data of the profile results are shown in Table 4.

[Table 4]

| Catalysts | Total desorbed $HN_3$(mmol/g) | Desorption temperature range(acid strength, mmol/g) | | |
| --- | --- | --- | --- | --- |
| | | 100 to 400□ (Weak desorption) | 401 to 650□ (Middle desorption) | 651 to 1000□ (Strong desorption) |
| $FeAlO_x$-10($Fe_2O_3$) | 0.34 | 0.07 | 0.24 | 0.03 |
| $FeAlO_x$-8 | 0.38 | 0.06 | 0.26 | 0.03 |
| $FeAlO_x$-5(catalyst of Example 1) | 2.35 | - | 2.35 | - |
| $FeAlO_x$-1 | 1.82 | - | 1.38 | 0.44 |
| $FeAlO_x$-0($Al_2O_3$) | 2.48 | - | 1.65 | 0.83 |
| Na-$Fe_3O_4$ | 0.09 | 0.05 | 0.04 | - |

In Table 4, the acid strength was classified into weak (100 to 400°C), medium (401 to 650°C), and strong (651 to 1000°C) regions. In the profile results, the acid strength (0.34 mmol/g) of FeAlO$_x$-10 (pure Fe$_2$O$_3$) was relatively low, while the acid strength (2.35 mmol/g) of the catalyst of Example 1 was relatively high. As the Al$_2$O$_3$ component increased to 100% (FeAlO$_x$-0), the acid strength increased to 2.48 mmol/g. In particular, in the catalyst of Example 1, FeAlO$_x$-1, and FeAlO$_x$-0 catalysts, the strength of most of the acidic sites decreased to a medium strength. The C-C coupling reaction was greatly influenced by the type and the strength of the acidic site. This ultimately determined the selectivity to the product in the course of CO$_2$ conversion via FT synthesis. In general, a strong acidic site (e.g., zeolite) has very good reactivity, such that long chain hydrocarbons may be excessively decomposed into gases having C$_1$ to C$_4$, whereas the weak acidic site is not very active in an oligomerization reaction. Therefore, the presence of a large number of strong or weak acidic sites is undesirable for selective production of C$_{5+}$ liquid hydrocarbons. The catalyst of Example 1 contains a relatively large amount of medium strength acidic sites (2.35 mmol/g) which may actively promote the C-C coupling reaction in producing the C$_{5+}$ liquid hydrocarbons and thus may be advantageous for CO$_2$ conversion. Al-rich catalysts (FeAlO$_x$-1 and FeAlO$_x$-0) contains a large number of medium-strength acidic sites. However, the content of compounds with iron oxide activity is close to zero (low-to-zero), thus limiting the RWGS reaction. This leads to low CO$_2$ conversion percentage and low selectivity to C$_{5+}$. Na-Fe$_3$O$_4$ contains very little medium strength acidic sites, such that the C-C coupling reaction may be promote , but the selectivity to the C$_{5+}$ liquid hydrocarbon is lowered.

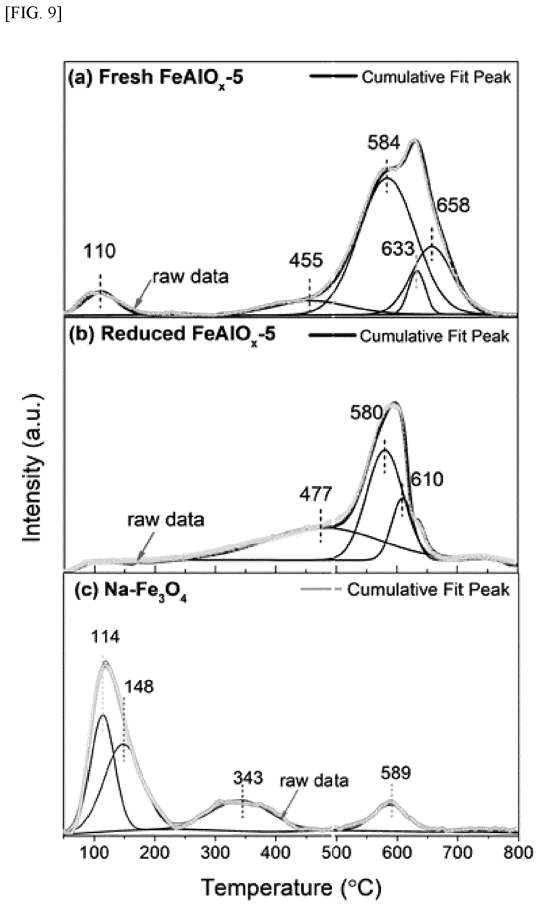

FIG. 9 shows the CO$_2$-TPD profile of (a) the catalyst of Example 1, (b) the reduced catalyst and (c) Na-Fe$_3$O$_4$. Surface basicity of the catalyst of Example 1 and Na-Fe$_3$O$_4$ catalyst was tested. To this end, CO$_2$ desorption analysis based on a temperature was performed. Detailed data thereof are shown in Table 5.

[Table 5]

| Catalysts | Total desorbed CO$_2$ (mmol/g) | Desorption temperature range(basicity, mmol/g) | | |
|---|---|---|---|---|
| | | 100 to 400□ (Weak desorption) | 401 to 6500□ (middle desorption) | 651 to 1000□ (Strong desorption) |
| Catalyst of Example 1 | 0.65 | 0.05 | 0.42 | 0.18 |
| Reduced catalyst | 0.62 | - | 0.48 | 0.14 |
| Na-Fe$_3$O$_4$ | 0.13 | 0.12 | 0.01 | - |

In Table 5, basic sites were classified into three types: weak, medium and strong sites based on a strength thereof. In the catalyst of Example 1 and the reduced catalyst, CO$_2$ desorption occurred mainly at the medium intensity. The total desorbed CO$_2$ of the catalyst of Example 1 and that of the reduced catalyst were approximately the same as each other (0.65 to 0.62 mmol/g). The catalyst of Example 1 and the reduced catalyst have a medium basic portion of 0.42 to 0.48 mmol/g and a strong basic portion of 0.14 to 0.18 mmol/g. In contrast, in Na-Fe$_3$O$_4$, there was almost no middle basic portion (0.01 mmol/g) and no peaks related to the strong basic site were observed, while a weak base site (0.12 mmol/g) was dominant. The middle and strong basic sites may play an important role in the formation of Fe-C bonds when reactants and intermediates are adsorbed onto the FeAlO$_x$ catalyst. The formation of olefins in the CO$_2$ conversion is highly dependent on the strength of the Fe-C bond. Therefore, as the Fe-C bond strength increases, the CO/H$_2$ coverage ratio on the catalyst surface increases, thereby to inhibit excessive hydrogenation of the CH$_2$-monomer, thereby preventing paraffin formation, thereby improving selectivity to olefins. In addition, in the catalyst of Example 1 and the reduced catalyst, the medium and strong strength basic sites may promote the formation of the Fe$_5$C$_2$ phase and thus improves the C-C coupling reaction.

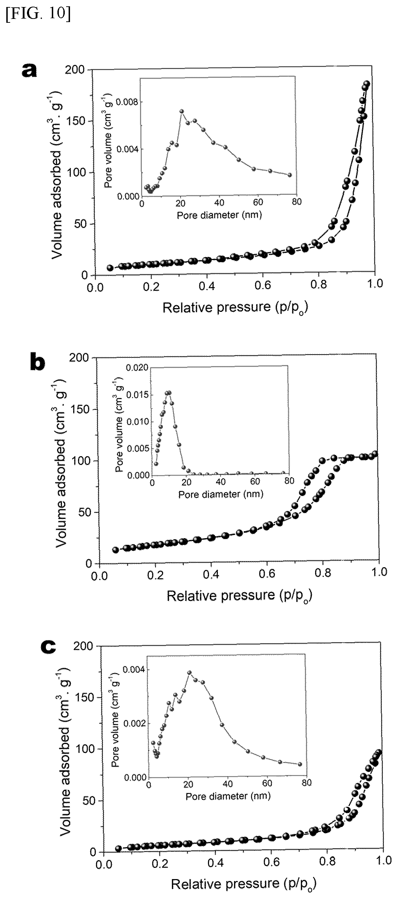

FIG. 10 shows the nitrogen (N$_2$) adsorption-desorption isotherms and pore size distributions of (a) the catalyst of Example 1, (b) reduced catalyst and (c) spent catalyst. Referring to Table 1, a BET (Brunauer-Emmett-Teller) surface area of the catalyst of Example 2 increased to 63.4m$^2$/g, and a pore size thereof decreased to 10.0nm, compared to the catalyst of Example 1 having BET surface area of 35.6m2/g and the pore size of 31.7nm. increase in the BET surface area in the reduced catalyst means an increased likelihood of exposing the active site to the reaction gas mixture, thus improving adsorption, and increasing the CO$_2$ conversion percentage and the product yield. An amount of residual Na in the FeAlO$_x$-5 catalyst as measured using ICP-OES (inductively coupled plasma-optical emission spectrometry) was 0.74 wt%. Since the presence of Na in FeAlO$_x$-5 increases the basicity in relation to the alkali metal, the heat of adsorption of CO increases and the heat of adsorption of H$_2$ deceases. The preferential adsorption of CO selectively promotes C-C coupling via FT synthesis and suppresses excessive hydrogenation in producing the olefins, thus making it difficult to form other products such as CH$_4$. In this connection, the Fe$_5$C$_2$ and AlO$_x$ active sites are adjacent to each other, such that the C$_2$ to C$_4$ olefins may be formed on FeAlO$_x$-5, and then may be re-adsorbed to the amorphous AlO$_x$ site, thus leading to the selective formation of C$_5$ hydrocarbons (77.0%) via the chain growth mechanism. To the contrary, the Na-Fe$_3$O$_4$ catalyst has high selectivity (34.5%) to C$_2$ to C$_4$ olefins, thus making the formation of C$_5$ hydrocarbons difficult due to a low percentage at which C$_2$ to C$_4$ olefins are resorbed thereto.

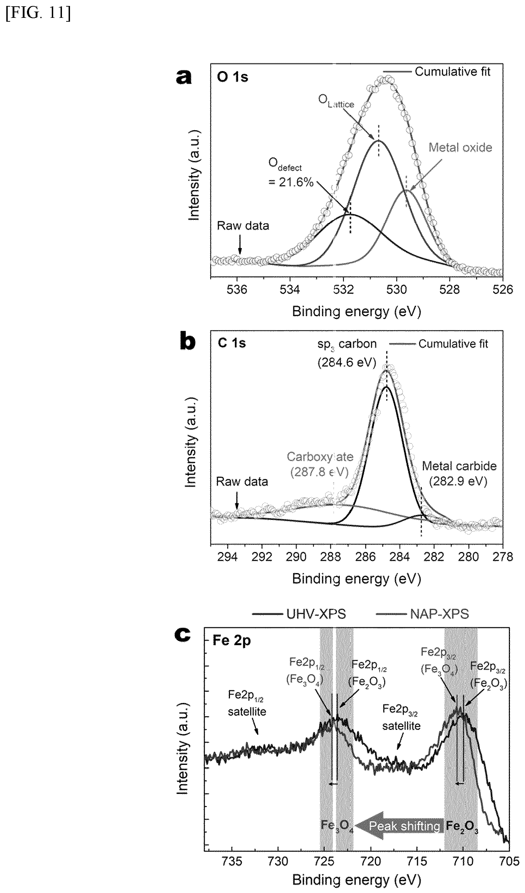

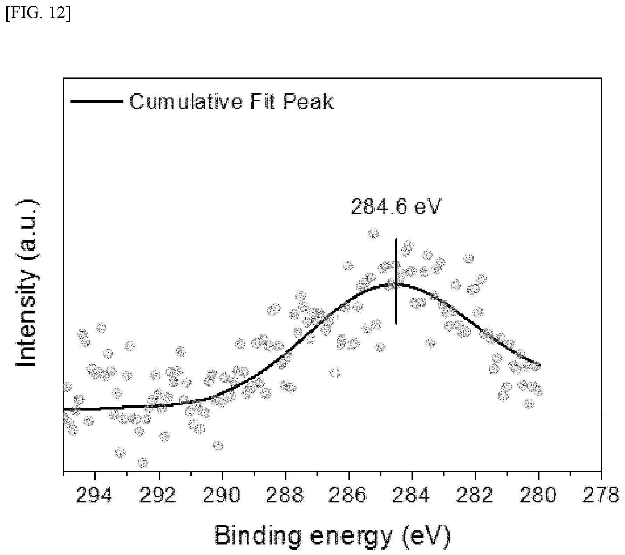

FIG. 11 is an in situ NAP-XPS result, and is intended to investigate the change of the chemical environment of the catalyst of Example 1 during the $CO_2$ conversion (330□, 0.1mbar $CO_2$, 0.1mbar $H_2$). In FIG. 11, (a) shows a high-resolution O 1s spectrum, (b) shows a high-resolution C 1s spectrum, and (c) shows a high-resolution Fe 2p spectrum. The data show that the catalyst of Example 1 is reduced during $CO_2$ conversion to create a significant amount of surface oxygen space (21.6%), which promotes the adsorption of $CO_2$ and H2 on the surface of the catalyst. As shown in (a) in FIG. 11, the O 1s spectrum may include peaks corresponding to oxygen defects at 531.7 eV, oxygen lattice atoms at 530.5 eV, and oxygen (O 1s) close to the metal-oxygen bond at 529.6 eV, respectively. Therefore, in the reaction condition, the catalyst of Example 1 has an oxygen defect to adsorb $CO_2$. Referring to FIG. 12 where a binding energy of C 1s was measured using UHV-XPS, the catalyst of Example 1 exhibited a weak peak at 284.6 eV. This means that sp$^3$ hybridized carbon is present. To the contrary, when a $CO_2/H_2$ mixture was used, two additional peaks appeared at 282.9 and 287.7 eV ((b) in FIG. 11) respectively, corresponding to metal carbide and metal carboxylate, respectively. The metal carbide phase may correspond to χ-$Fe_5C_2$ and involve in a C-C coupling reaction. The XPS data in the Fe 2p region obtained under UHV and NAP conditions are shown in (c) in FIG. 1. Under the UHV condition, binding energy peaks (Fe 2p$_{3/2}$ and Fe 2p$_{1/2}$ occurring at 709.8 and 723.5 eV, respectively) corresponding to $Fe_2O_3$ appear. To the contrary, when there is a reaction gas mixture, there is a peak shift to higher binding energy (Fe 2p$_{3/2}$ and Fe 2p$_{1/2}$ shift to 711.0 and 724.2 eV, respectively), which means that in the RWGS process of hydrogenating $CO_2$ using the catalyst of Example 1, $Fe_3O_4$ is formed. Thus, it may be identified that an active site for $CO_2$ hydrogenation is present in the catalyst of Example 1.

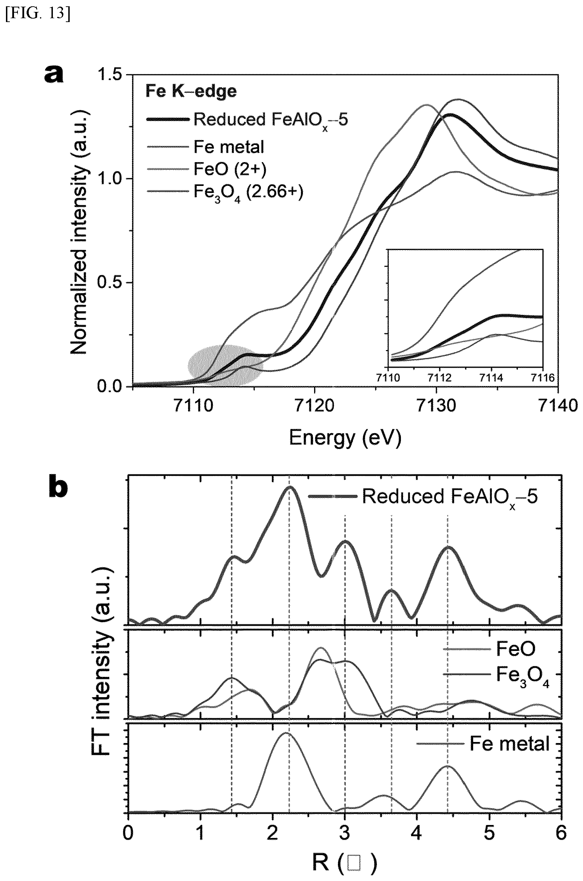

FIG. 13 shows the XAS of the catalyst of Example 2, in which the catalyst of Example 2 was tested with different references (Fe foil, FeO, and $Fe_3O_4$) to further investigate the properties of the active site. In FIG. 13, (a) shows the X-ray absorption near edge structure (XANES) spectrum, which shows the standard spectrum of each of $Fe_3O_4$ and the Fe foil. Based on (a) in FIG. 13, it may be identified that $Fe_2O_3$ is reduced to $Fe_3O_4$ and a metallic α-Fe phase is formed. (b) in FIG. 13 shows the Fourier transform intensity of the extended X-ray absorption fine structure (EXAFS) spectrum. This shows that a first hybrid shell corresponds to a Fe-O pathway of $Fe_3O_4$, while a second hybrid shell corresponds to a Fe-Fe pathway of metallic α-iron together with a BCC structure. A third shell corresponds to a Fe-Fe pathway on $Fe_3O_4$. The presence of $Fe_3O_4$ and metallic iron phases in the reduced catalyst indicates that a reduction site in the RWGS reaction is present. This is in agreement with the NAP-XPS and XRD results.

In performing the carbon dioxide conversion using the catalysts of Examples, a test was performed to find out an optimum condition. Process parameters including the $H_2/CO_2$ ratio, the reaction pressure, and the space velocity were varied.

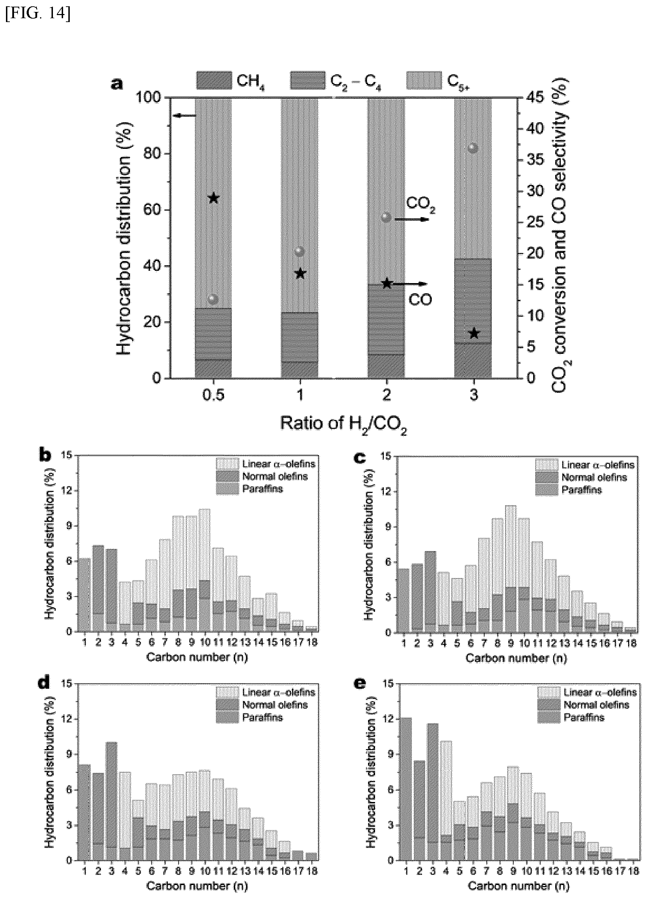

FIG. 14 shows the result of performing carbon dioxide conversion with the catalyst of Example 1 while setting various $H_2/CO_2$ ratios. In FIG. 14, (a) shows the $CO_2$ conversion and selectivity to CO at each of the ratios, and (b) to (d) show a distribution of a final product when the ratio of $H_2/CO_2$ is 0.5, 1, 2, and 3, respectively. Increasing the $H_2/CO_2$ ratio from 0.5:1 to 3:1 greatly increased the $CO_2$ conversion from 12.5% to 36.8%, and sharply decreased selectivity to CO from 28.9% to 7.2%. As a result, when the $H_2/CO_2$ ratio increased from 0.5:1 to 3:1, the $C_{5+}$ yield increased from 6.7% to 19.7%. This indicates that a larger amount of $CO_2$ is converted to liquid hydrocarbons via the FT mechanism in the presence of sufficient $H_2$. In addition, when $H_2$ was sufficiently present, the formation of oxygenated compounds (e.g., ethanol, acetic acid, 1-butanol) was suppressed from 3.2% to 0.7%. Referring to (b) to (d) in FIG. 14, it may be identified that when the $H_2/CO_2$ ratio is reduced to 3:1, formation of paraffin is preferred via hydrogenation of olefins. In this connection, an O/P (oxygen/paraffin) ratio of $C_2^+$ was 5.1 when the $H_2/CO_2$ ratio was 1:0. However, the O/P (oxygen/paraffin) ratio of $C_2^+$ decreased to 2.3 when the $H_2/CO_2$ ratio was 3:1.

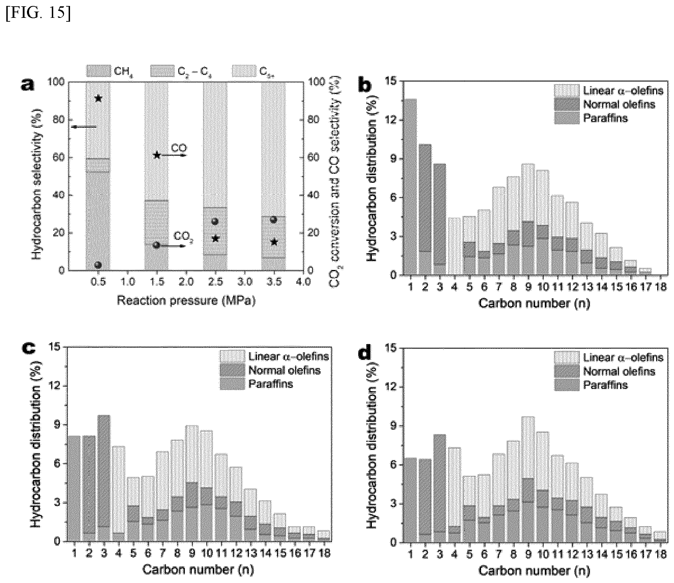

FIG. 15 shows the result of carbon dioxide conversion with various pressure settings. The pressure was set in a range of 0.5 to 3.5 MPa (0.5 MPa unit) at 330°C at a ratio of $H_2/CO_2$ of 2:1. When the reaction pressure was increased from 0.5 to 2.5 MPa, the $CO_2$ conversion percentage increased noticeably from 2.6% to 25.7%, and $C_{5+}$ selectivity increased from 40.8 to 66.8%. Increasing the pressure to 3.5 MPa further increased the $CO_2$ conversion percentage to 26.7% and the $C_{5+}$ selectivity (that is, selectivity to $C_{5+}$) to 71.7%. A product distribution was slightly changed while the pressure changes. This indicates that the pressure does not have a significant effect on the product.

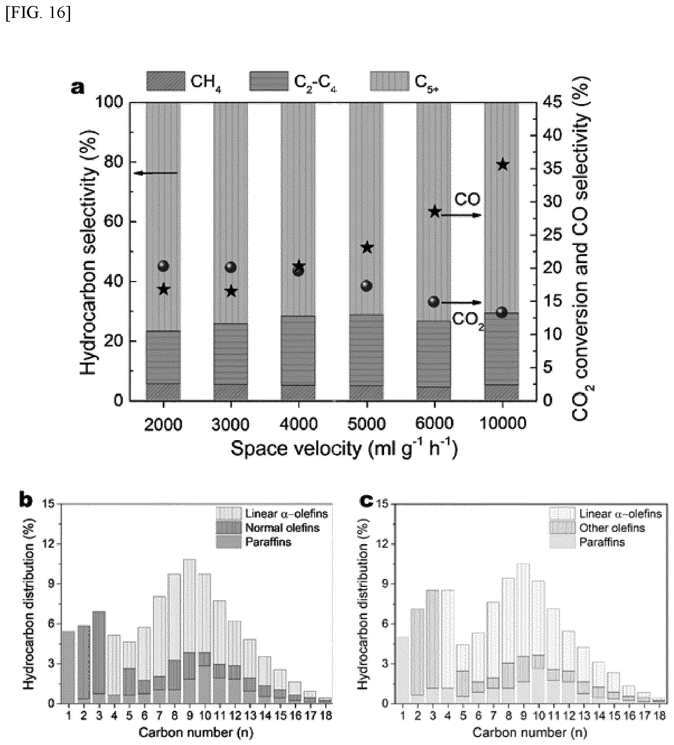

FIG. 16 shows the results of carbon dioxide conversion with various space velocity settings. The $H_2/CO_2$ ratio was fixed to 1:1 and the space velocity of the $CO_2$ and $H_2$ gas mixture was increased from 2000 to 10000ml/g·h. When the space velocity becomes 10000ml/g·h, the $CO_2$ conversion percentage decreased from 20.2% to 13.%, and CO selectivity increased from 16.8% to 35.6%. However, the $C_{5+}$ selectivity changed in a range of 71.0 to 77.0%, and thus there was no significant change in the $C_{5+}$ selectivity.

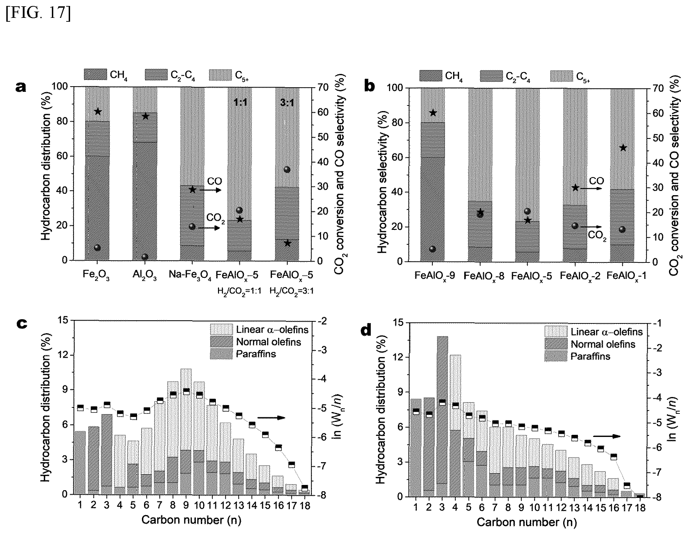

FIG. 17 shows the $CO_2$ conversion percentage, the CO selectivity, and the hydrocarbon selectivity (including CO) after performing the $CO_2$ hydrogenation with various catalysts. As shown in (a) in FIG. 17, when the $H_2/CO_2$ ratio is 1:1 and the $CO_2$ conversion percentage is 20.2%, the selectivity of the catalyst of Example 1 with a 1:1 Fe/Al ratio to $C_{5+}$ liquid hydrocarbon was 77.0%(excluding CO). However, the selectivity thereof to each of CO (16.8%), CH4 (5.4%), and $C_2$ to $C_4$ (17.6%) was reduced. In addition, the yield of $C_{5+}$ including CO was 12.6%. However, increasing the $H_2/CO_2$ ratio to 3:1 significantly increased the $CO_2$ conversion percentage to 36.8%. In this case, the selectivity thereof to CO was reduced to 7.2%. The selectivity to $C_{5+}$ hydrogenation decreased slightly to 57.8% (excluding CO), but the overall $C_{5+}$ yield (including

CO) increased to 19.7%. When the $H_2/CO_2$ ratio was 1:1 or 3:1, the high $CO_2$ conversion percentage was achieved. The $C_{5+}$ yield was higher, compared to that of the conventional catalyst Na-$Fe_3O_4$, which has a higher $CO_2$ conversion percentage.

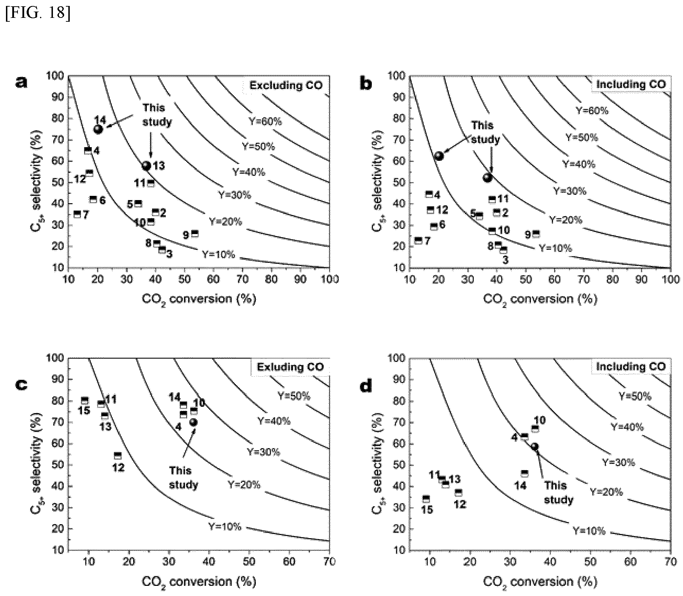

(a) in FIG. 18 as a comparison result between the present disclosure and previous studies in terms of the $CO_2$ conversion percentage and the $C_{5+}$ selectivity (where a single metal oxide catalyst is used and CO is excluded) indicates that the best $C_{5+}$ selectivity may be obtained using a $FeAlO_x$-5 catalyst at 1:1 of a ratio of $H_2/CO_2$ without the use of an additional C-C coupling catalyst (e.g., zeolite), while CO is not taken into account in the $C_{5+}$ selectivity calculation (as in the most of previous experiments). In addition, (b) in FIG. 18 (including CO and using a single metal oxide catalyst) indicates that when CO is considered in the $C_{5+}$ selectivity calculation (when a perfect carbon balance for all $CO_2$ conversion products is provided), the catalyst of Example 1 has an unprecedented high $C_{5+}$ liquid hydrocarbon yield (19.7%) compared to those of the conventional catalysts (3.0 to 16.1%).

A formula for calculating the $C_{5+}$ selectivity when the CO is not considered (Equation 1) and a formula for calculating the $C_{5+}$ selectivity when the CO is considered (Equation 2) are as follows:

[Equation 1]

$$\text{HC selectivity (C} - \text{mol\%) excluding CO} = \frac{\text{Moles of } C_i \text{ hydrocarbon} \times i}{\sum_{i=1}^{n} \text{Moles of } C_i \text{ hydrocarbons} \times i} \times 100\%$$

[Equation 2]

$$\text{HC selectivity (C} - \text{mol\%) including CO} = \frac{\text{Moles of } C_i \text{ hydrocarbon} \times i}{CO_{2\text{ in}} - CO_{2\text{ out}}} \times 100\%$$

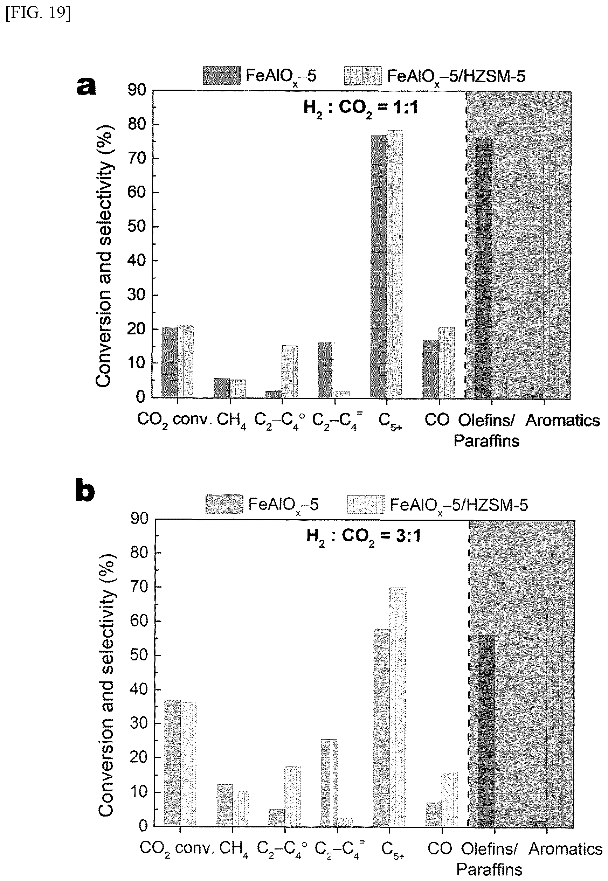

Due to the low $C_{5+}$ selectivity of the single type catalyst, most of the previous studies have employed a metal oxide/zeolite composite catalyst. Therefore, in order to see whether the catalyst properties are improved when zeolite is mixed with the catalyst of Example 1, $FeAlO_x$-5/HZSM-5 (the catalyst of Example 2) was synthesized based on 80 of a $SiO_2/Al_2O_3$ ratio. The $CO_2$ conversion was performed using the $FeAlO_x$-5/HZSM-5/. The results are shown in FIG. 19. Referring to (a) in FIG. 19, the $CO_2$ conversion percentage of the $FeAlO_x$-5/HZSM-5 composite catalyst and the selectivity of the $FeAlO_x$-5/HZSM-5 composite catalyst to each of CO, $CH_4$, and $C_{5+}$ hydrocarbons were similar to those of the single catalyst of the catalyst of Example 1 at a 1:1 ratio of $H_2/CO_2$. When the ratio of $H_2/CO_2$ was increased to 3:1, the selectivity of $FeAlO_x$-5/HZSM-5 to $C_{5+}$ increased from 57.8% to 70.0% (excluding CO), and the $C_{5+}$ yield slightly increased from 19.7 to 21.6% (including CO), compared to that of the $FeAlO_x$-5 single catalyst. When the conventional catalysts were combined with zeolite, the $C_{5+}$ yield increased from 11.7% (Na-$Fe_3O_4$) to 21.2% (Na-$Fe_3O_4$/HZSM-5) and from 16.1% (ZnFe$O_x$) to 24.3% (ZnFe$O_x$/HZSM-5). However, when the catalyst of Example 1 of the present disclosure was combined with zeolite, a significant effect on the $C_{5+}$ yield was not achieved.

In (a) in FIG. 17, the catalyst characteristics of $FeAlO_x$ were compared with those of single metal catalysts ($Fe_2O_3$, $Al_2O_3$, and Na-$Fe_3O_4$). This is intended to investigate the role of $FeAlO_x$-5 in producing $C_{5+}$ hydrocarbons at high yield without incorporating the zeolite therein. The $Fe_2O_3$ and $Al_2O_3$ catalysts exhibited very low $CO_2$ conversion percentages (4.8% and 1.2%, respectively). This means that the $CO_2$ activity was not significantly exhibited due to the $Fe_2O_3$ and $Al_2O_3$ phases. Regarding the Na-$Fe_3O_4$ catalyst, the selectivity thereof to $C_{5+}$ (excluding CO) was much lower (57.1%) than that of the catalyst of Example 1. However, the selectivity thereof to $C_2$ to $C_4$ (excluding CO) and the selectivity thereof CO were much greater (34.5% and 28.6%, respectively) than those of the catalyst of Example 1 (17.6% and 16.8%, respectively).

(b) in FIG. 17 shows the results of evaluating the $CO_2$ conversion percentage and the products selectivity of the $FeAlO_x$ catalyst having a Fe/Al ratio of 1 to 9 based on contents of Fe and Al. When the ratio of Fe/Al decreased from 9 to 5, the $CO_2$ conversion percentage increased from 4.8% to 20.2% and the $C_{5+}$ selectivity increased from 20.0% to 77.0%, but the CO selectivity decreased from 60.0% to 16.8%. To the contrary, when the Fe/Al ratio was further reduced from 5 to 1, the $CO_2$ conversion percentage decreased from 20.2% to 13.0%, and the $C_{5+}$ selectivity decreased from 77.0% to 58.4%, whereas the CO selectivity was significantly increased from 16.8% to 46.2%. This indicates that the ratio of Fe/Al plays an important role in determining the $CO_2$ conversion percentage and the products selectivity.

In (c) and (d) in FIG. 17, the product distributions resulting from the catalyst reactions of the catalyst of Example 1 and the Na-$Fe_3O_4$ catalyst are quantified using an effective carbon number method. Regarding the catalyst of Example 1, the selectivity thereof to $C_2^+$ olefin was high and was 78.5%, and $C_{4+}$ olefins accounted for 66.8%. In particular, the selectivity thereof to $C_{4+}$ LAOs was 52.4%. The selectivity thereof to liquid olefin ($C_5$ to $C_{18}$) was 62.3%. In this connection, the liquid LAOs accounted for 47.9%. Most of the LAOs in the liquid product were $C_6$ to $C_{11}$, which is an industrially important compound in the production of industrial plasticizers and lubricants. In the reaction using the catalyst of Example 1, the yields of $C_{4+}$ LAOs and total $C_{4+}$ olefins were 10.6% and 13.5%, respectively. These values are unprecedentedly high compared to those of previous studies. To the contrary, regarding Na-$Fe_3O_4$, the selectivity thereto of $C_{4+}$ LAOs was much lower than that of $FeAlO_x$-5 (4.8%) ((d) in FIG. 17). Thus, it may be identified that the catalysts in Examples of the present disclosure improves the chain growth reaction and the LAO production.

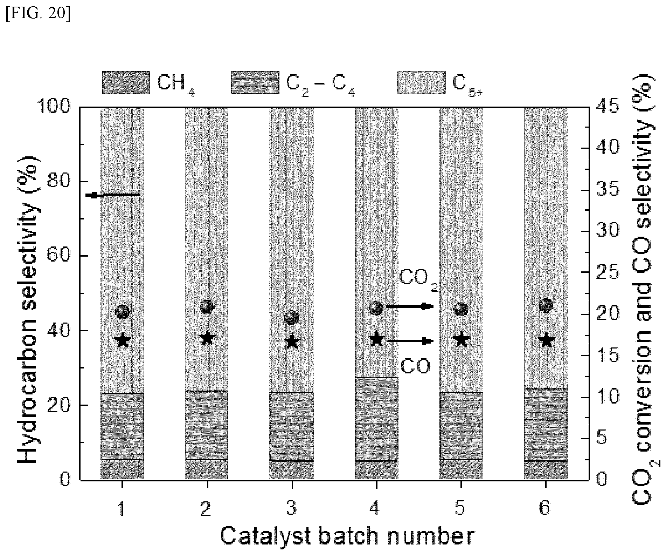

FIG. 20 shows the result of evaluating the reproducibility of the catalyst of Example 1. $CO_2$ hydrogenation and product synthesis were tested in different batches at different times. The reaction condition was as follows: a space velocity of 3000 mL/g·h ($CO_2$ = 1000 mL/g·h, $H_2$ = 2000 mL/g·h) at 330°C and 3.5 MPa at a ratio of $H_2/CO_2$ of 2:1. As shown in FIG. 20, it may be seen that the reactivity of and selectivity to all components are almost unaffected by the time and are reproduced uniformly and ideally.

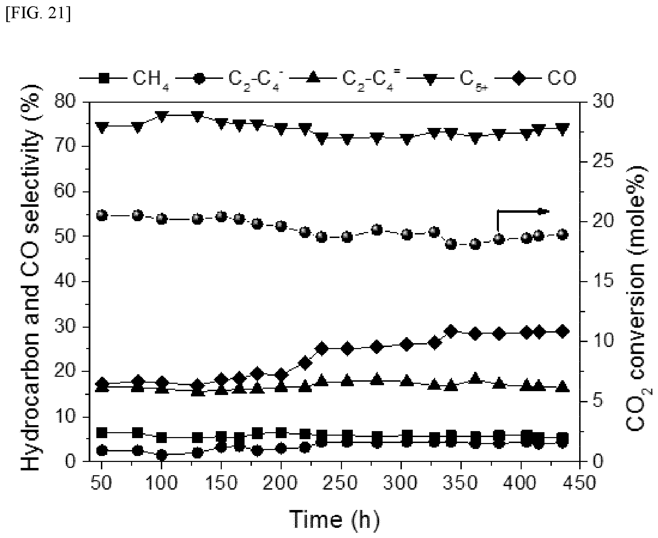

FIG. 21 shows the result of evaluating the stability of the catalyst of Example 1. The reaction condition was as follows: a space velocity of 2000 mL/g·h ($CO_2$ = 1000 mL/g·h, $H_2$ = 1000 mL/g·h) at 330°C and 3.5 MPa at a ratio of $H_2/CO_2$ of 1:1. Until the reaction time 450 hours have lapsed, the $CO_2$ conversion percentage and the selectivity to each hydrocarbon rem are maintained at a similar level on the stream. However. The selectivity to CO increased slightly from 17.2% to 28.9% after the reaction time 450 hours haver lapsed. This resulted in a slight decrease in the yield of $C_{5+}$ liquid hydrocarbons from 11.9% to 10.1% at the end of the reaction on the stream for 450 hours. During the reaction using the catalyst of Example 1 for 450 hours, there was a slight change in the selectivity, but no noticeable change thereof was detected. Thus, it was identified that the catalyst of Example 1 had stable catalyst activity for a long time.

As described above, the present disclosure is described with reference to the drawings. However, the present disclosure is not limited to the embodiments and drawings disclosed in the present specification. It will be apparent that various modifications may be made thereto by those skilled in the art within the scope of the present claims. Furthermore, although the effect resulting from the features of the present disclosure has not been explicitly described in the description of the embodiments of the present disclosure, it is obvious that a predictable effect resulting from the features of the present disclosure should be recognized.

## Claims

1. A method for converting carbon dioxide using a catalyst for converting carbon dioxide, the catalyst being **characterized in that** the catalyst has a composition of $Fe_xAl_yO_z$, wherein x is a real number of 0.1 inclusive to 0.8 inclusive, y is a real number of 0.2 inclusive to 0.9 inclusive, and z is a real number of 0.5 inclusive to 3 inclusive; the method being **characterized in that** the method comprises:

   reacting the catalyst with hydrogen gas to adsorb hydrogen thereon;
   injecting a gas mixture of carbon dioxide and hydrogen such that the gas mixture reacts with the hydrogen-adsorbed catalyst;
   wherein the catalyst contains a crystallized $Fe_2O_3$ phase and an amorphous aluminum oxide, wherein the $Fe_2O_3$ phase and the aluminum oxide are adjacent to each other.

2. The method of claim 2, wherein the at% ratio of Fe and Al is 50:50.

3. The method of claim 1, wherein the $Fe_2O_3$ phase is fired at 400 to 1000°C to act as a basic site.

4. The method of claim 1, further comprising an alkali metal which includes at least one selected from the group consisting of sodium (Na), potassium (K), rubidium (Rb), and cesium (Cs).

5. The method of claim 4, wherein an amount of the alkali metal is in a range of 0.5 to 10 wt% with respect to a total weight of the catalyst.

6. The method of claim 1, wherein hydrogen desorption occurs on the aluminum oxide.

7. The method of claim 1, wherein the aluminum oxide is fired at 400 to 1000°C to act as an acidic site.

8. The method of claim 1, wherein the catalyst is combined with zeolite to form a composite.

9. The method of claim 1, wherein a mixing ratio of the carbon dioxide and the hydrogen in the gas mixture is in a range of 3:1 to 1:3.

10. The method of claim 1, wherein the gas mixture is injected at a space velocity of a range of 2000 to 10000 mL/g·h.

**11.** The method of claim 1, wherein a reaction pressure by the gas mixture is in a range of 1.5 to 3.5 MPa.

**12.** The method of claim 1, wherein the method is carried out at a temperature of 300 to 500°C.

**Patentansprüche**

**1.** Verfahren zum Umwandeln von Kohlendioxid unter Verwendung eines Katalysators zum Umwandeln von Kohlendioxid, wobei der Katalysator **dadurch gekennzeichnet ist, dass** der Katalysator eine Zusammensetzung von $Fe_xAl_yO_z$ aufweist, wobei x eine reelle Zahl von einschließlich 0,1 bis einschließlich 0,8 ist, y eine reelle Zahl von einschließlich 0,2 bis einschließlich 0,9 ist und z eine reelle Zahl von einschließlich 0,5 bis einschließlich 3 ist; wobei das Verfahren **dadurch gekennzeichnet ist, dass** das Verfahren umfasst:

Reaktion des Katalysators mit Wasserstoffgas, um Wasserstoff daran zu adsorbieren;
Injizieren einer Gasmischung aus Kohlendioxid und Wasserstoff, so dass die Gasmischung mit dem Wasserstoff-adsorbierten Katalysator reagiert;
wobei der Katalysator eine kristallisierte $Fe_2O_3$ -Phase und ein amorphes Aluminiumoxid enthält, wobei die $Fe_2O_3$ -Phase und das Aluminiumoxid einander benachbart sind.

**2.** Verfahren nach Anspruch 1, wobei das At%-Verhältnis von Fe und Al 50:50 beträgt.

**3.** Verfahren nach Anspruch 1, wobei die $Fe_2O_3$ -Phase bei 400 bis 1000 °C gebrannt wird, um als eine basische Stelle zu wirken.

**4.** Verfahren nach Anspruch 1, ferner umfassend ein Alkalimetall, das mindestens eines aus der Gruppe bestehend aus Natrium (Na), Kalium (K), Rubidium (Rb) und Cäsium (Cs) enthält.

**5.** Verfahren nach Anspruch 4, wobei eine Menge des Alkalimetalls in einem Bereich von 0,5 bis 10 Gew.-%, bezogen auf ein Gesamtgewicht des Katalysators, liegt.

**6.** Verfahren nach Anspruch 1, wobei eine Wasserstoffdesorption auf dem Aluminiumoxid stattfindet.

**7.** Verfahren nach Anspruch 1, wobei das Aluminiumoxid bei 400 bis 1000 °C gebrannt wird, um als eine saure Stelle zu wirken.

**8.** Verfahren nach Anspruch 1, wobei der Katalysator mit Zeolith kombiniert wird, um einen Verbundstoff zu bilden.

**9.** Verfahren nach Anspruch 1, wobei ein Mischungsverhältnis des Kohlendioxids und des Wasserstoffs in der Gasmischung in einem Bereich von 3:1 bis 1:3 liegt.

**10.** Verfahren nach Anspruch 1, wobei die Gasmischung mit einer Raumgeschwindigkeit in einem Bereich von 2000 bis 10000 ml/g·h injiziert wird.

**11.** Verfahren nach Anspruch 1, wobei ein Reaktionsdruck durch die Gasmischung in einem Bereich von 1,5 bis 3,5 MPa liegt.

**12.** Verfahren nach Anspruch 1, wobei das Verfahren bei einer Temperatur von 300 bis 500 °C durchgeführt wird.

**Revendications**

**1.** Procédé de conversion de dioxyde de carbone utilisant un catalyseur pour convertir le dioxyde de carbone, le catalyseur étant **caractérisé en ce que** le catalyseur a une composition de $Fe_xAl_yO_z$ dans lequel x est un nombre réel de 0,1 inclusif à 0,8 inclusif, y est un nombre réel de 0,2 inclusif à 0,9 inclusif et z est un nombre réel de 0,5 inclusif à 3 inclusif ;
le procédé étant **caractérisé en ce que** le procédé comprend :

la réaction du catalyseur avec du gaz hydrogène pour adsorber l'hydrogène dessus ;

l'injection d'un mélange gazeux de dioxyde de carbone et d'hydrogène de telle manière que le mélange gazeux réagit avec le catalyseur avec l'hydrogène adsorbé ;
le catalyseur contenant une phase de $Fe_2O_3$ cristallisé et un oxyde d'aluminium amorphe, la phase de $Fe_2O_3$ et l'oxyde d'aluminium étant adjacents l'un à l'autre.

2. Procédé selon la revendication 1, dans lequel le rapport de % at de Fe et d'Al est de 50 : 50.

3. Procédé selon la revendication 1, dans lequel la phase de $Fe_2O_3$ est allumée à 400 à 1 000 °C pour agir comme un site basique.

4. Procédé selon la revendication 1, comprenant en outre un métal alcalin qui comprend au moins un choisi dans le groupe constitué par le sodium (Na), le potassium (K), le rubidium (Rb) et le césium (Cs).

5. Procédé selon la revendication 4, dans lequel une quantité du métal alcalin se situe dans une plage de 0,5 à 10 % en poids par rapport à un poids total du catalyseur.

6. Procédé selon la revendication 1, dans lequel la désorption d'hydrogène se produit sur l'oxyde d'aluminium.

7. Procédé selon la revendication 1, dans lequel l'oxyde d'aluminium est allumé à 400 à 1 000 °C pour agir comme un site acide.

8. Procédé selon la revendication 1, dans lequel le catalyseur est combiné avec de la zéolite pour former un composite.

9. Procédé selon la revendication 1, dans lequel un rapport de mélange du dioxyde de carbone et de l'hydrogène dans le mélange gazeux se situe dans une plage de 3 : 1 à 1 : 3.

10. Procédé selon la revendication 1, dans lequel le mélange gazeux est injecté à une vitesse spatiale de 2 000 à 10 000 mL/g·h.

11. Procédé selon la revendication 1, dans lequel une pression de réaction par le mélange gazeux se situe dans une plage de 1,5 à 3,5 MPa.

12. Procédé selon la revendication 1, dans lequel le procédé est réalisé à une température de 300 à 500 °C.

[FIG. 1]

[FIG. 2]

[FIG. 3]

[FIG. 4]

[FIG. 5]

[FIG. 6]

[FIG. 7]

[FIG. 8]

[FIG. 9]

[FIG. 10]

[FIG. 11]

[FIG. 12]

[FIG. 13]

[FIG. 14]

[FIG. 15]

[FIG. 16]

[FIG. 17]

[FIG. 18]

[FIG. 19]

[FIG. 20]

[FIG. 21]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014111919 A2

**Non-patent literature cited in the description**

- **MIRON V. LANDAU et al.** *CHEMSUSCHEM*, 23 February 2014, vol. 7, ISSN 1864-5631, 785-794